# FASCICULE DE BREVET EUROPEEN

(11) **EP 1 351 648 B1**
(45) Date de publication et mention de la délivrance du brevet: **16.04.2014**
(21) Numéro de dépôt: 01996347.9
(22) Date de dépôt: 20.11.2001
(51) Int. Cl.: A61K 47/48, A61K 38/17, A61P 43/00

(54) **VECTEURS DE TRANSPORT A TRAVERS UN EPITHELIUM A JONCTIONS SERREES**
VEKTOREN FÜR TRANSPORT DURCH EIN EPITHELIUM MIT DICHTEN VERBINDUNGSSTELLEN
CARRIER VECTORS THROUGH AN EPITHELIUM WITH TIGHT JUNCTIONS

(30) Priorité: 20.11.2000 FR 0014945
(43) Date de publication de la demande: 15.10.2003
(73) Titulaire: CENTRE NATIONAL DE LA RECHERCHE SCIENTIFIQUE (CNRS), 75016 Paris (FR)
(72) Inventeur: JOLIOT, Alain, F-75012 Paris (FR); DUPONT, Edmond, F-75018 Paris (FR); PROCHIANTZ, Alain, F-75006 Paris (FR)
(74) Mandataire: Vialle-Presles, Marie José
(86) Numéro de dépôt international: PCT/FR2001/003631
(87) Numéro de publication internationale: WO 2002/039947

(56) Documents cités:
- WO-A-00/29427
- WO-A-00/58488
- WO-A-00/62067
- WO-A-01/13957
- WO-A-01/38547
- WO-A-99/05302
- DEROSSI D (REPRINT) ET AL: "Trojan peptides: the penetratin system for intracellular delivery" TRENDS IN CELL BIOLOGY, vol. 8, février 1998 (1998-02), pages 84-87, XP002122131 ISSN: 0962-8924
- SCHWARZE S ET AL: "In vivo protein transduction: delivery of a biologically active protein into the mouse" SCIENCE, AMERICAN ASSOCIATION FOR THE ADVANCEMENT OF SCIENCE, US, vol. 285, no. 5433, 3 septembre 1999 (1999-09-03), pages 1569-1572, XP002140133 ISSN: 0036-8075
- BELLET-AMALRIC E ET AL: "INTERACTION OF THE THIRD HELIX OF ANTENNAPEDIA HOMEODOMAIN AND A PHOSPHOLIPID MONOLAYER, STUDIED BY ELLIPSOMETRY AND PM-IRRAS AT THEAIR-WATER INTERFACE" BIOCHIMICA ET BIOPHYSICA ACTA, AMSTERDAM, NL, vol. 1467, no. 1, 31 juillet 2000 (2000-07-31), pages 131-143, XP001004550 ISSN: 0006-3002
- ROUSSELLE C ET AL: "NEW ADVANCES IN THE TRANSPORT OF DOXORUBICIN THROUGH THE BLOOD-BRAIN BARRIER BY A PEPTIDE VECTOR-MEDIATED STRATEGY" MOLECULAR PHARMACOLOGY, BALTIMORE, MD, US, vol. 57, no. 4, avril 2000 (2000-04), pages 679-686, XP001004535 ISSN: 0026-895X
- FISCHER P M ET AL: "STRUCTURE-ACTIVITY RELATIONSHIP OF TRUNCATED AND SUBSTITUTED ANALOGUES OF THE INTRACELLULAR DELIVERY VECTOR PENETRATIN" JOURNAL OF PEPTIDE RESEARCH, MUNKSGAARD INTERNATIONAL PUBLISHERS, COPENHAGEN, DK, vol. 55, no. 2, février 2000 (2000-02), pages 163-172, XP000899124 ISSN: 1397-002X
- THOREN P E G ET AL: "THE ANTENNAPEDIA PEPTIDE PENETRATIN TRANSLOCATES ACROSS LIPID BILAYERS - THE FIRST DIRECT OBSERVATION" FEBS LETTERS, ELSEVIER SCIENCE PUBLISHERS, AMSTERDAM, NL, vol. 482, no. 3, 6 octobre 2000 (2000-10-06), pages 265-268, XP001004487 ISSN: 0014-5793
- PROCHIANTZ A: "Getting hydrophilic compounds into cells: lessons from homeopeptides" CURRENT OPINION IN NEUROBIOLOGY, LONDON, GB, vol. 6, no. 5, 1 octobre 1996 (1996-10-01), pages 629-634, XP002087113 ISSN: 0959-4388
- JAEGER E A: "Small synthetic peptides homologous to segments of occludin impair tight junction resealing in a Ca2+-switch assay in A6 cell monolayers" MOLECULAR BIOLOGY OF THE CELL, vol. 8, suppl., novembre 1997 (1997-11), page 205A, abrégé no. 1189 XP002099341 & 37th Annual Meeting of the American Society for Cell Biology; Washington, D.C., USA; December 13-17, 1997 ISSN: 1059-1524
- DATHE, MARGITTA ET AL: "Peptide Helicity and Membrane Surface Charge Modulate the Balance of Electrostatic and Hydrophobic Interactions with Lipid Bilayers and Biological Membranes" BIOCHEMISTRY, 1996, VOL. 35, NO. 38, PAGE(S) 12612-12622, XP002176664
- OEHLKE, J. ET AL: "Rapid translocation of amphipathic alpha-helical and beta-sheet-forming peptides through plasma membranes of endothelial cells" PEPT. SCI.: PRESENT FUTURE, PROC. INT. PEPT. SYMP., 1ST, 1999, VOL. MEETING DATE 1997, 782-783. EDITOR(S): SHIMONISHI, YASUTSUGU. PUBLISHER: KLUWER, DORDRECHT, NETH., XP001016351
- SCHELLER, ANNE ET AL: "Structural requirements for cellular uptake of.alpha.-helical amphipathic peptides" J. PEPT. SCI., 1999, VOL. 5, NO. 4, PAGE(S) 185-194, XP001016347

## Description

La présente invention est relative à de nouveaux vecteurs capables de transporter des molécules actives à travers la barrière hémato-méningée et, plus généralement, à travers des épithéliums formant des barrières étanches entre deux compartiments.

La barrière hémato-méningée (BHE) a pour fonction de préserver le cerveau des variations de la composition sanguine, et de protéger les cellules nerveuses de substances potentiellement toxiques. Elle réside au niveau de l'endothélium des vaisseaux capillaires irriguant le système nerveux central. Cet endothélium présente des propriétés particulières qui le différencient de celui des autres capillaires de l'organisme. Il est dépourvu des pores que l'on trouve dans un grand nombre d'autres capillaires. En outre, les cellules qui le constituent sont étroitement jointes les unes aux autres. Ces " jonctions serrées " possèdent en outre une résistance électrique élevée (de l'ordre de 1000 ohms/cm²), et constituent donc un obstacle efficace même vis-à-vis de la diffusion passive des ions.

La barrière hémato-méningée constitue donc un filtre très sélectif, qui ne laisse naturellement passer qu'un petit nombre de substances. Il s'agit principalement :
- de molécules lipophiles de faible poids moléculaire, qui peuvent traverser la barrière hémato-méningée par diffusion passive ;
- de molécules hydrophiles de faible poids moléculaire, constituant des nutriments essentiels pour le fonctionnement des cellules nerveuses (notamment sucres, acides aminés, neuropeptides, vitamines, etc). Ces molécules traversent la barrière hémato-méningée par l'intermédiaire de transporteurs spécifiques ;
- de molécules de poids moléculaire élevé (par exemple des hormones polypeptidiques), qui traversent la barrière hémato-méningée après fixation à leurs récepteurs spécifiques présents à la surface des cellules endothéliales.

L'imperméabilité de la barrière hémato-méningée constitue un obstacle majeur à la pénétration cérébrale de nombreux médicaments.

Pour y remédier, plusieurs approches ont été proposées :
1) L'administration directe de la molécule souhaitée au niveau du système nerveux central, par perfusion ou injection intrathécale. Cette méthode présente l'inconvénient d'être extrêmement invasive et délicate à mettre en oeuvre. Une approche voisine est constituée par la greffe, dans le système nerveux central, de cellules génétiquement modifiées pour produire la substance à administrer, agissant ainsi à la façon de " minipompes " pour délivrer celle-ci *in situ*. Malgré son intérêt potentiel, cette approche demeurera limitée aux drogues pouvant être synthétisées et sécrétées par une cellule.
2) L'ouverture momentanée de la BHE, en général provoquée par choc osmotique, ou par l'action de certaines substances (par exemple bradykinine ou angiotensine), qui ouvrent transitoirement les jonctions serrées en interagissant avec le domaine extracellulaire de certains de leur composants. Cette approche présente l'inconvénient d'être non-sélective, et de permettre le passage de nombreuses autre molécules présentes dans le sang en même temps que la molécule à transporter.
3) L'utilisation des mécanismes naturels de passage à travers la barrière hémato-méningée. La diffusion passive ne peut toutefois être utilisée que pour des substances de faible poids moléculaire, lipophiles, ou pouvant être rendues lipophiles sans pour autant modifier leurs propriétés pharmacologiques. Les transporteurs spécifiques ne peuvent être utilisés que pour des molécules de structure suffisamment proche de celle des substances naturellement transportées par leur intermédiaire. Dans le cas des récepteurs spécifiques, il a été proposé de conjuguer des ligands naturels de ces récepteurs avec la molécule à transporter ; un inconvénient majeur de cette approche réside dans l'activité biologique propre de ces ligands, qui peut entraîner des effets secondaires plus ou moins gênants.

Récemment, il a été proposé d'utiliser, comme vecteurs de transport à travers la barrière hémato-méningée, des peptides transducteurs. Les peptides transducteurs sont des peptides comprenant une séquence dénommée " domaine de transduction " leur conférant la capacité de pénétrer à l'intérieur d'une cellule vivante, indépendamment de la présence de transporteurs ou de récepteurs spécifiques.

Des articles de revue concernant les peptides transducteurs ont été publiés récemment par LIDGREN et al., [TiPS, 21, 99-102, (2000)] ; SCHWARZE et DOWDY [TiPS, 21, 45-48, (2000)] ; SCHWARZE et al. [Trends Cell. Biol., 10, 290-295, (2000)] ; PROCHIANTZ [Current Opinion in Cell Biology, 12, 400-406, (2000)].

A titre d'exemples de peptides transducteurs, on citera en particulier :
- les pénétratines, qui sont des peptides dérivés de la troisième hélice d'un homéodomaine ; des peptides de la famille des pénétratines sont décrits par exemple dans les publications de JOLIOT et al. [Proc. Natl. Acad. Sci. USA, 88, 1864-1868, (1991)] ; DEROSSI et al. [J. Biol. Chem., 269, 14, 10444-10450, (1994)] ; BRUGIDOU et al. [Biophys. Biochem. Res. Com., 214, 685-693, (1995)], ainsi que dans le Brevet US 5888762, le Brevet US.6080724, ou la Demande PCT WO/00/01417.
- les peptides dérivés de la protéine Tat de HIV1, et en particulier du fragment 48-60 de ladite protéine ; de tels peptides sont décrits par exemple par FAWELL et al. [Proc. Natl. Acad. Sci. USA., 91, 664-668, (1994)] ou par VIVES et al. [J. Biol. Chem., 272, 16010-16017, (1997)].
- les peptides dérivés de la protéine VP22 de HSV ; de tels peptides sont décrits par exemple par ELLIOTT et O'HARE [Cell, 88, 223-233, (1997)]. Ces peptides, qui contiennent des signaux d'adressage nucléaire, s'accumulent dans le noyau après leur pénétration dans la cellule ; la Demande PCT WO 00/58488 propose de fusionner ces peptides avec des séquences d'export nucléaire afin de les relocaliser dans le cytoplasme lorsque leur accumulation dans le noyau n'est pas souhaitée.
- des peptides dérivés d'une séquence signal, éventuellement conjuguée à une séquence de localisation nucléaire ; de tels peptides sont décrits par exemple par LIN et al. [J. Biol. Chem., 270, 14255-14258, (1995) ; J. Biol. Chem., 271, 5305-5308, (1996)], LIU et al. [Proc. Natl. Acad. Sci. USA, 93, 11819-11824, (1996)], MORRIS et al. [Nucleic Acids Res., 25, 2730-2736, (1997)], CHALOIN et al. [Biochemistry, 36, 11179-11187, (1997) ; Biochem. Biophys. Res. Commun., 243, 601-608, (1998)], ZHANG et al. [Proc. Natl. Acad. Sci. USA, 95, 9184-9189, (1998)].
- les transportanes qui sont dérivés d'une fusion entre une portion d'un neuropeptide, la galanine, et un peptide du venin de guêpe [POOGA et al., FASEB J., 12, 67-77, (1998) ; Ann. New York Acad. Sci., 863, 450-453, (1998)].

SCHWARZE et al. [Science, 285, 1569-1572, (1999)] ont observé que la β-galactosidase couplée à un domaine de transduction de la protéine Tat, pouvait être détectée, après administration par voie intra-péritonéale, dans tous les tissus de l'organisme, y compris le cerveau.

ROUSSELLE et al. [Mol Pharmacol, 57, 679-686, (2000)] rapportent également que le couplage de la doxorubicine avec la D-pénétratine ou le peptide SynB1 accroît son passage à travers la barrière hémato-méningée.

Bien que l'utilisation de peptides transducteurs paraisse augmenter le passage à travers la barrière hémato-méningée celui-ci demeure toutefois très faible.

D'autre part, lors de travaux antérieurs, l'équipe des Inventeurs a mis en évidence, dans la protéine à homéodomaine Engrailed, l'existence d'une séquence d'export nucléaire participant à la sortie de cette protéine du noyau et à son passage dans un compartiment de sécrétion [JOLIOT et al., Curr Biol, 8, 856-863, (1998) ; MAIZEL et al., Development, 126, 3183-3190, (1999)]. Cette séquence diffère, par sa structure et sa position dans la molécule, d'une séquence de sécrétion classique de type peptide signal.

Les Inventeurs ont alors recherché si cette séquence d'export nucléaire était fonctionnelle en dehors du contexte de la protéine Engrailed entière. Dans ce but, ils ont comparé les propriétés de pénétration et de distribution intracellulaire d'un peptide comprenant uniquement un domaine de transduction (constitué par une pénétratine, en l'occurrence la troisième hélice du peptide pAntp), et d'un peptide (NES-Pénét) comprenant, outre ce domaine de transduction, la séquence d'export nucléaire de la protéine Engrailed. Ils ont constaté qu'après pénétration dans les cellules à 37°C le peptide comprenant uniquement le domaine de transduction est localisé dans le noyau, et de manière diffuse, dans le cytoplasme, alors que le peptide comprenant également la séquence d'export nucléaire est principalement localisé dans un compartiment vésiculaire du cytoplasme. Après pénétration dans les cellules à 4°C le peptide comprenant uniquement le domaine de transduction ainsi que celui comprenant également la séquence d'export nucléaire restent localisés dans le noyau. Si les cellules sont alors portées à 37°C, on observe le passage du peptide comprenant le domaine de transduction et la séquence d'export nucléaire dans le compartiment vésiculaire, ce qui traduit un mécanisme d'export nucléaire énergie-dépendant.

Les Inventeurs ont également recherché si l'addition de la séquence d'export nucléaire modifiait la capacité d'interaction de la pénétratine avec le compartiment vésiculaire. Dans ce but, ils ont incubé chacun des 2 peptides avec des fractions membranaires purifiées, et les ont traités avec de la trypsine. Ils ont alors observé que le peptide contenant le domaine de transduction seul (pénétratine) était presque totalement dégradé par la trypsine, alors que le peptide comprenant le domaine de transduction et la séquence d'export nucléaire (NES-Penet) était protégé contre l'action de la trypsine. La même expérience de fractionnement et de protection a été menée après internalisation par les cellules vivantes ; la dégradation de la pénétratine et la protection du peptide NES-Penet ont également été observées. Le fait que la pénétratine incubée avec les membranes seules ne soit pas protégée suggère que le rôle de la séquence d'export nucléaire n'est pas limité à la sortie du noyau mais qu'elle permet aussi l'accès du peptide à un compartiment intracisternal, et son accumulation dans celui-ci.

Afin de déterminer si le passage dans le compartiment vésiculaire pouvait résulter en une externalisation effective du peptide, et si cette externalisation était susceptible de faciliter le passage à travers un épithélium à jonctions serrées, les Inventeurs ont utilisé un modèle expérimental constitué par 2 compartiments séparés par une membrane microporeuse recouverte d'un tapis cellulaire reproduisant un épithélium à jonctions serrées. L'un des compartiments est en contact avec le pôle apical des cellules, et l'autre avec leur pôle baso-latéral. Ils ont ainsi constaté que lorsque l'on ajoute dans le compartiment baso-latéral le peptide pénétratine comprenant uniquement le domaine de transduction, on observe l'entrée de ce peptide dans les cellules, et un faible passage vers le compartiment apical. Lorsque le même peptide est ajouté dans le compartiment apical, il entre également dans les cellules ; par contre, on n'observe aucun passage vers le compartiment baso-latéral.

Les mêmes expériences ont été effectuées avec le peptide NES-Penet cas, le passage du compartiment baso-latéral au compartiment apical est très fortement augmenté, alors que le passage du compartiment apical au compartiment baso-latéral demeure faible ou inexistant.

En outre, les Inventeurs ont comparé, à 4°C et 37°C l'entrée et la sortie des peptides pénétratine et NES-Penet placés dans l'un ou l'autre des deux compartiments. Ils ont observé que l'entrée de l'un ou l'autre de ces peptides se produit à 4°C comme à 37°C, mais que la sortie de NES-Penet est bloquée à 4°C, ce qui confirme que les mécanismes d'entrée et de sortie sont distincts.

Il apparaît donc que l'association d'une séquence d'export nucléaire avec un domaine de transduction permet d'augmenter l'efficacité du transport à travers un épithélium à jonction serrées, dans le sens baso-latéral/apical.

La présente invention a pour objet l'utilisation d'un peptide comprenant un domaine de transduction dérivé de la troisième hélice d'un homéodomaine et une séquence d'export nucléaire, en tant que vecteur pour transporter une molécule d'intérêt à travers un épithélium à jonctions serrées, notamment à travers la barrière hémato-méningée et le plexus choroïde.

En particulier la présente invention a pour objet l'utilisation d'au moins une séquence d'acides aminés constituant un domaine de transduction dérivé de la troisième hélice d'un homéodomaine et d'au moins une séquence d'acides aminés constituant une séquence d'export nucléaire, pour l'obtention d'un vecteur destiné au transport d'une molécule d'intérêt diagnostique ou thérapeutique à travers un épithélium à jonctions serrées, notamment à travers la barrière hémato-méningée et le plexus choroïde.

Des domaines de transduction dérivés de la troisième hélice d'un homéodomaine, utilisables pour la mise en oeuvre de la présente invention sont connus en eux-mêmes et sont notamment décrits dans les publications de l'art antérieur mentionnées ci-dessus.

Ledit domaine de transduction peut ou non comprendre une séquence de localisation nucléaire (NLS).

On définit par " séquence de localisation nucléaire " une séquence qui lorsqu'elle est présente dans un domaine de transduction, permet l'accès de celui-ci au noyau après son entrée dans la cellule.

A titre d'exemples de peptides constituant des domaines de transduction dérivés de la troisième hélice d'un homéodomaine, et comprenant une séquence de localisation nucléaire, on citera les pénétratines.

A titre d'exemples de peptides constituant des domaines de transduction dérivés de la troisième hélice d'un homéodomaine, et ne comprenant pas de séquence de localisation nucléaire, on citera certains variants des pénétratines [DEROSSI et al., J. Biol. Chem., 271, 18188-18193, (1996)].

Dans le cas où le domaine de transduction ne comprend pas de séquence de localisation nucléaire, on peut éventuellement, si on le souhaite, lui associer une séquence de localisation nucléaire hétérologue.

Par : " séquence d'export nucléaire " (NES), on entend toute séquence peptidique capable de conférer à un peptide comprenant ladite séquence la capacité de sortir du noyau cellulaire et de gagner un compartiment de sécrétion.

Des séquences d'export nucléaire connues en elles-mêmes sont utilisables pour la mise en oeuvre de la présente invention. 3 types de séquences d'export nucléaire ont été décrits à l'heure actuelle (pour revue cf. par exemple NAKIELNY et DREYFUSS, Current Opinion in Cell Biology 9, 420-429, 1997) : les séquences d'export nucléaire dites " riches en leucine ", qui sont des séquences d'environ 10 acides aminés comprenant une proportion importante d'acides aminés hydrophobes aliphatiques (leucine ou isoleucine, et également valine ou alanine), qui sont présentes dans un grand nombre de protéines, parmi lesquelles les protéines à homéodomaine ; la séquence M9 de hnRNP A1 [MICHAEL et al., Cell, 83, 415-422, (1995)] ; la séquence KNS (pour : " hnRNP K nuclear shuttling domain ") de hnRNP K [MICHAEL et al., EMBO J., 16, 3587-3598, (1997)].

Selon un mode de mise en oeuvre préféré de la présente invention, on utilisera une séquence d'export nucléaire riche en leucine, et notamment une séquence d'export nucléaire d'une protéine à homéodomaine. Des séquences d'export nucléaire des protéines à homéodomaine pouvant en particulier être utilisées pour la mise en oeuvre de la présente invention, sont des séquences de 10 à 15 acides aminés environ, localisées, dans ces protéines, à l'extrémité N-terminale de la 3^{ème} hélice, ou recouvrant partiellement celle-ci. On peut, à titre d'exemple, utiliser une séquence d'export nucléaire de la protéine " Engrailed ", comprenant au moins la séquence (code 1 lettre) AQELGLNESQI (SEQ ID NO: 2), et avantageusement, au moins la séquence (code 1 lettre) SLAQELGLNERQIKI (SEQ ID NO: 3).

La séquence d'export nucléaire et le domaine de transduction peuvent être directement adjacents, séparés par une autre séquence d'acides aminés, ou se recouvrir au moins partiellement.

La présente invention peut être mise en oeuvre pour transporter à travers un épithélium à jonctions serrées, et notamment à travers la barrière hémato-méningée, toute molécule d'intérêt diagnostique ou thérapeutique pouvant être transportée à l'intérieur d'une cellule vivante par un peptide transducteur, y compris une protéine à homédomaine. Elle peut ainsi être mise en oeuvre notamment pour le transport d'acides nucléiques, de polypeptides, de peptides/acides nucléiques, ou d'analogues de nucléotides utilisés notamment comme agents antinéoplasiques.

Pour la mise en oeuvre de la présente invention, la molécule à transporter peut être fixée à n'importe quel niveau du peptide vecteur, à condition que cette fixation ne modifie pas les fonctionnalités du domaine de transduction ou du séquence d'export nucléaire. On peut par exemple fixer ladite molécule à l'extrémité N-terminale, à l'extrémité C-terminale, ou à l'intérieur dudit peptide, par exemple entre le domaine de transduction et la séquence d'export nucléaire.

La conjugaison entre la molécule à transporter et le peptide vecteur peut être effectuée directement, ou par l'intermédiaire d'un lieur, par les mêmes méthodes que celles utilisées pour la conjugaison à un peptide transducteur. Différentes méthodes appropriées sont connues en elles-mêmes, et sont notamment décrites dans les publications de l'art antérieur mentionnées ci-dessus.

La présente invention sera mieux comprise à l'aide du complément de description qui va suivre, qui se réfère à des exemples, illustrant le passage d'un peptide comprenant un domaine de transduction dérivé de la troisième hélice d'un homéodomaine et un domaine d'export nucléaire à travers un épithélium à jonctions serrées.

### EXEMPLE 1 : LOCALISATION INTRACELLULAIRE D'UN PEPTIDE COMPRENANT LE DOMAINE DE TRANSDUCTION DU PEPTIDE pANTP ET LA SEQUENCE D'EXPORT NUCLEAIRE DE LA PROTEINE ENGRAILED.

On synthétise le peptide biotinylé :
Biot-QSLAQELGLNERQIKIWFQNRRMKWKK-COOH (SEQ ID NO: 4).

Ce peptide (dénommé ci-après NES-Penet) comprend, avec un recouvrement partiel, une séquence :
QSLAQELGLNERQIKI (SEQ ID NO: 5)
contenant la séquence d'export nucléaire de la protéine Engrailed,
et une séquence pénétratine :
RQIKIWFQNRRMKWKK (SEQ ID NO: 6)
constituée par l'hélice 3 du peptide pAntp (homéodomaine de la protéine Antennapedia de drosophile).

Il est en outre marqué à son extrémité N-terminale par une biotine fixée par l'intermédiaire d'un bras aminopentanoïque.

A titre de témoin, le peptide biotinylé :
Biot-RQIKIWFQNRRMKWKK-COOH (dénommé ci-après Penet-1)
ne comprenant que la séquence pénétratine a également été synthétisé.

Chacun des 2 peptides a été incubé, à la concentration de 10 µM, avec des cellules cérébrales d'embryon de rat (E17, 10⁷ cellules/ml) en suspension, pendant 1 heure à 37°C.

Les cellules ont été lysées et fractionnées selon le protocole suivant :
On lyse les cellules dans 200 µl de tampon Hepes 10 mM, MgCl2 3 mM, pH 7 contenant un cocktail d'inhibiteurs de protéases. La lyse est effectuée par dix aller-retour au travers d'une aiguille G26 montée sur une seringue de 1 ml.

Après centrifugation des extraits (150,000g 1 heure, 4°C) au travers d'un coussin de saccharose discontinu 0,25 M et 1,7 M les noyaux sont récupérés dans le culot et la fraction membranaire à l'interface 0,25 M/1,7 M.

Une partie de chacune ces fractions a ensuite été traitée à la trypsine selon le protocole suivant : incubation en tampon phosphate (PBS) 1 heure, 4°C, en présence de trypsine 100 µg/ml ; arrêt de la réaction par addition d'acide trichloracétique (TCA) 10% (vol/vol) à 100°C pendant 5 min.

Les fractions, non-traitées ou traitées à la trypsine, ont ensuite été analysées par électrophorèse en conditions dénaturantes sur gel de polyacrylamide 22% en présence de SDS.

La révélation des peptides Penet-1 et NES-Penet est effectuée à la streptavidine peroxydase selon le protocole décrit par DEROSSI et al. [J. Biol. Chem., 271, 18188-18193, (1996)].

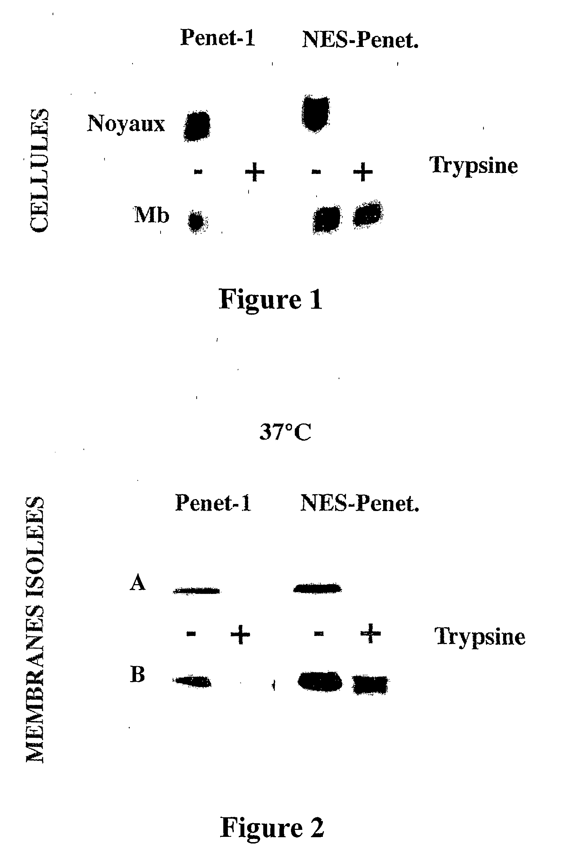

Les résultats sont illustrés par la Figure 1.

En l'absence de traitement par la trypsine (-), on observe une association des peptides Penet-1 et NES-Penet avec la fraction nucléaire (Noyaux) et avec la fraction membranaire (Mb).

Le traitement par la trypsine (+) induit la dégradation du peptide Penet-1 dans la fraction nucléaire et la fraction membranaire. En revanche, dans le cas du peptide NES-Penet, on n'observe pas de dégradation dans la fraction membranaire, et une protection partielle contre la dégradation dans la fraction nucléaire. Cette protection reflète probablement une légère contamination de cette fraction par des membranes périnucléaires.

Dans une 2^{ème} série d'expériences, les cellules en culture ont été lysées et les fractions membranaires purifiées selon le protocole décrit ci-dessus, sans incubation préalable avec les peptides.

Chacun des 2 peptides Penet-1 et NES-Penet a été incubé, à la concentration de 10 µM, avec la préparation de fractions membranaires issue de 10⁷ neurones (comme ci-dessus), pendant 1 heure à 37°C, puis traité à la trypsine selon le protocole décrit ci-dessus.

Après incubation, le mélange peptide/préparation membranaire a été traité à la trypsine selon le protocole décrit ci-dessus.

Parallèlement, chacun des 2 peptides Penet-1 et NES-Penet a été traité à la trypsine, dans les mêmes conditions, sans incubation préalable avec la préparation membranaire.

Les lysats trypsiques ont été analysés par électrophorèse comme décrit ci-dessus.

Les résultats sont illustrés par la Figure 2.

En l'absence d'incubation avec la préparation membranaire (A), on observe une dégradation totale des peptides Penet-1 et NES-Penet après traitement par la trypsine (+).

Après incubation avec la préparation membranaire (B), on observe, après traitement par la trypsine (+), une protection partielle du peptide Penet-1 (la quantité de peptide protégé pouvant être évaluée à moins de 30%), et une protection quasi-totale du peptide NES-Penet.

Ces résultats montrent qu'indépendamment de la présence de noyaux, la séquence d'export nucléaire favorise l'accès du peptide à un compartiment intra-membranaire, et/ou son maintien dans ce compartiment.

### EXEMPLE 2 : PASSAGE D'UN PEPTIDE COMPRENANT LE DOMAINE DE TRANSDUCTION DU PEPTIDE pANTP ET LA SEQUENCE D'EXPORT NUCLEAIRE DE LA PROTEINE ENGRAILED A TRAVERS UN EPITHELIUM A JONCTIONS SERREES.

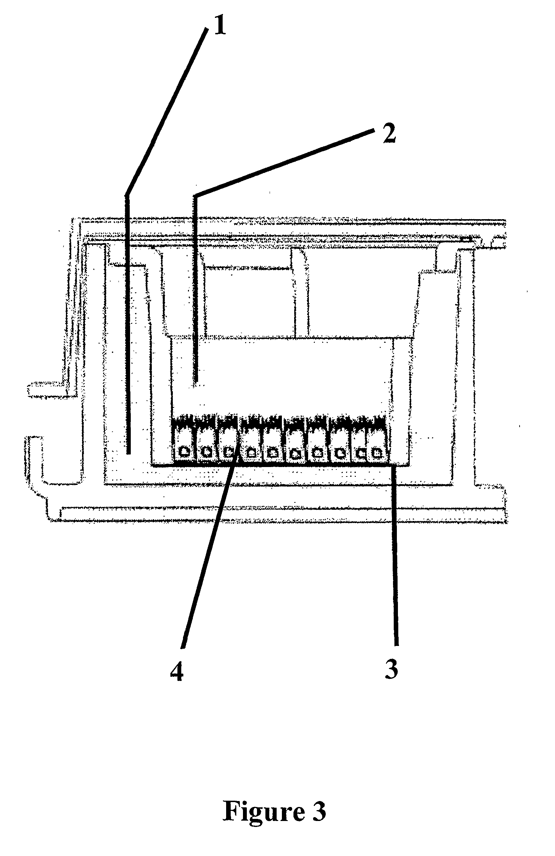

Le passage des peptides Penet et NES-Penet à travers un épithélium à jonctions serrées a été évalué à l'aide du dispositif (système TRANSWELL, CORNING-COSTAR) représenté sur la Figure 3.

Ce dispositif comprend deux compartiments (1, 2) séparés par une membrane microporeuse en polyester (3) sur laquelle un épithélium (4) constitué de cellules MDCK (Madin-Darby Canine Kidney, lignée de cellules de rein de chien) a été établi. Les cellules sont ensemencées et poussent jusqu'à confluence.

Le compartiment (1) au contact de la membrane microporeuse et du pôle baso-latéral des cellules est dénommé compartiment baso-latéral. Le compartiment (2), au contact du pôle apical des cellules est dénommé compartiment apical.

Chacun des compartiments est rempli de milieu dont la constitution est la suivante : Minimum Essential Medium with Earle Salt (MEM), Glutamine 2 mM, Hepes pH 7 2 mM, Pénicilline 5 U/ml, Streptomycine 5 µg/ml, additionné d'un mélange d'inhibiteurs des protéases.

Pour chaque expérience, l'intégrité des jonctions serrées est contrôlée par la mesure de la résistance entre les deux compartiments (R>2000Ω/cm²).

Par ailleurs l'étanchéité de l'épithélium a également été vérifiée en ajoutant de l'inuline tritiée (6,5x10⁵ cpm/compartiment), soit au compartiment apical, soit au compartiment baso-latéral, et en mesurant la radioactivité dans l'autre compartiment 180 minutes plus tard. Dans ces conditions, aucun passage de l'inuline tritiée n'a été observé, dans le sens apical/baso-latéral, ou dans le sens baso-latéral/apical.

Le peptide biotinylé Penet-1 ou le peptide biotinylé NES-Penet, utilisés chacun à la concentration de 10 µM, sont placés soit dans le compartiment apical, soit dans le compartiment baso-latéral). L'autre compartiment contient de la streptavidine, à une concentration de 10 µM permettant la capture du peptide biotinylé. Après 30 minutes d'incubation à 37°C, ou à 4°C, le milieu du compartiment de départ, et le milieu du compartiment d'arrivée sont prélevés.

Les peptides sont précipités par addition d'acide trichloracétique (TCA) à 10%, et repris dans du milieu de Laemmli bouillant. Les cellules sont rincées dans une solution de NaCl à 0,5M, puis lysées dans du milieu de Laemmli bouillant. La présence des peptides Penet-1 ou NES-Penet dans le compartiment de départ, dans le compartiment d'arrivée, ou à l'intérieur des cellules, est détectée par révélation à la streptavidine-peroxydase comme indiqué à l'Exemple 1 ci-dessus.

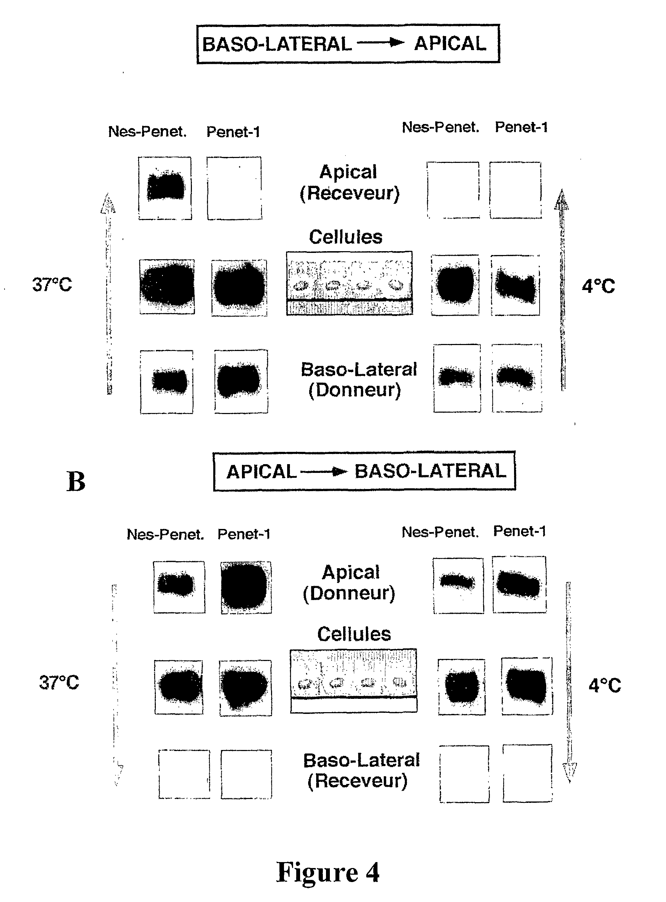

Les résultats sont illustrés par les Figures 4A et 4B.

La Figure 4A représente l'analyse électrophorétique des peptides NES-Penet et Penet-1 après 30 minutes d'incubation, lorsque le compartiment de départ est le compartiment baso-latéral. A 37°C (panneaux de gauche), on observe une pénétration des 2 peptides dans les cellules, et un très faible passage du peptide Penet-1 dans le compartiment apical ; en revanche le passage du peptide NES-Penet dans le compartiment apical est beaucoup plus important. On observe en outre que le passage ne s'accompagne d'aucune dégradation du peptide. A 4°C (panneaux de droite), on observe également une pénétration des 2 peptides dans les cellules ; en revanche aucun des 2 peptides ne passe dans le compartiment apical.

La Figure 4B représente l'analyse électrophorétique des peptides NES-Penet et Penet-1 après 30 minutes d'incubation, lorsque le compartiment de départ est le compartiment apical. A 37°C comme à 4°C, on observe une pénétration des 2 peptides dans les cellules, mais aucun passage dans le compartiment baso-latéral.

Lorsque les mêmes expériences sont effectuées en présence de sérum de veau, à une concentration de 10% dans le compartiment de départ on observe des résultats identiques, ce qui montre que le passage n'est pas bloqué par des composants sériques.

Ces résultats démontrent que les deux peptides peuvent être internalisés par les deux faces de l'épithélium, mais que la présence de la séquence d'export nucléaire augmente sensiblement le passage du peptide Nes-Penet à travers l'épithélium, et que ce passage est polarisé et se fait uniquement dans le sens baso-latéral/apical.

En outre, la température est sans effet sur l'entrée (elle se produit à 4°C et 37°C) alors que la sortie est température dépendante (elle est bloquée à 4°C), ce qui montre que ces 2 phénomènes correspondent à des processus distincts.

Dans une autre série d'expérimentations, l'effet de la brefeldine A (BFA) qui bloque la sécrétion protéique faisant intervenir l'appareil de Golgi, sur le passage du peptide NES-Penet a été étudié.

Le peptide biotinylé NES-Penet (10 µM) est incubé dans le compartiment basolatéral, dans les conditions décrites ci-dessus, pendant une heure à 4°C ou à 37°C, en l'absence ou en présence de brefeldine A (10 µM).

A la fin de l'incubation, les contenus du compartiment basolatéral et du compartiment apical, et celui des cellules sont analysés comme décrit ci-dessus pour détecter la présence du peptide NES-Penet.

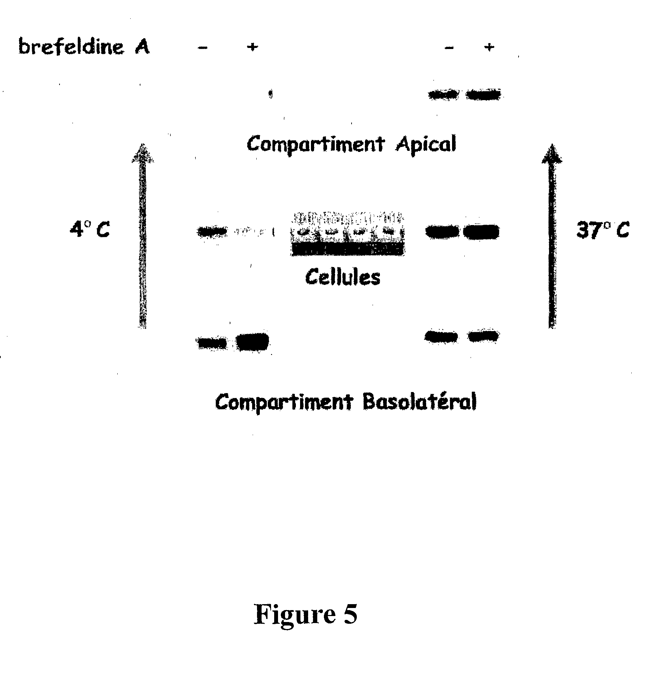

Les résultats sont illustrés par la Figure 5.

Ces résultats confirment que le peptide NES-Penet entre à 4°C (panneaux de gauche) mais ne ressort pas à cette température, que ce soit en présence (+) ou en l'absence (-) de brefeldine A. En revanche, à 37°C (panneaux de droite), on observe la sortie du peptide NES-Penet en présence (+) ou en l'absence (-) de brefeldine A.

Ces résultats montrent que la traversée de l'épithélium par le peptide Nes-Penet n'implique pas un passage par l'appareil de Golgi.

### EXEMPLE 3 : PASSAGE IN VIVO DE NES-PENET A TRAVERS LA BARRIERE HEMATOMENINGEE.

Le peptide NES-Penet biotinylé (50 µg/50 µl de PBS) ou du PBS (50 µl) sont injectés dans la veine de la queue d'une souris; celle-ci est sacrifiée une heure après l'injection. Le cerveau est prélevé et fixé au paraformaldéhyde 4%, et cryoprotégé par immersion pendant une nuit à 4°C dans une solution de PBS contenant 20% de saccharose. Des coupes frontales du cerveau (10 µM d'épaisseur) sont ensuite effectuées au cryostat. Le peptide est ensuite révélé sur ces coupes à l'aide d'un complexe streptavidine-fluorescéine, et la fluorescence est analysée par microscopie confocale.

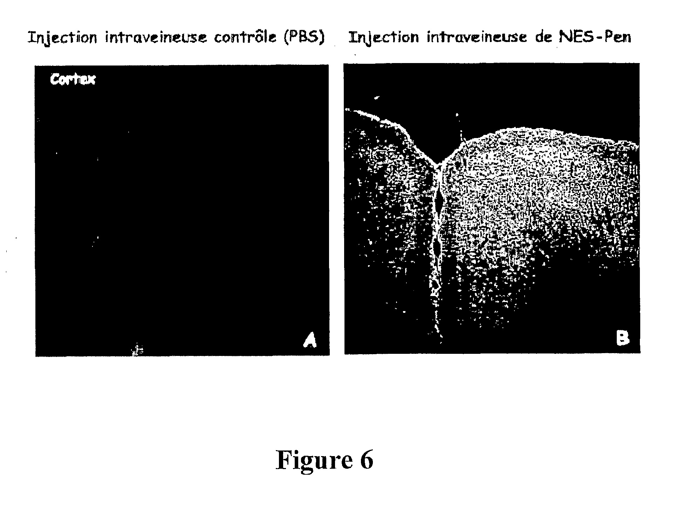

Les résultats sont illustrés par la Figure 6 (A : PBS ; B : peptide NES-Penet). Ces résultats montrent que le peptide NES-Penet biotinylé passe la barrière hémato-méningée de façon très importante, et parvient dans le cortex et les régions sub-corticales.

### EXEMPLE 4 : INFLUENCE DE LA SEQUENCE D'EXPORT NUCLEAIRE SUR LA LOCALISATION SUBCELLULAIRE ET LE PASSAGE TRANS-EPITHELIAL DE PEPTIDES CONTENANT DES DOMAINES DE TRANSDUCTION DE DIFFERENTS TYPES.

La localisation intracellulaire, et le passage à travers un épithélium à jonctions serrées du peptide NES-Penet décrits ci-dessus ont été comparés avec ceux d'un peptide, dénommé ci-après NES-Pen3P, comprenant la séquence d'export nucléaire de la protéine Engrailed décrite à l'Exemple 1, et une séquence pénétratine (dénommée ci-après Pen3P), dérivée de l'hélice 3 du peptide pAntp par substitution des résidus Ile, Gln et Lys situés respectivement en positions 45, 50 et 55 de l'homéodomaine Antp, par 3 résidus, proline. Le peptide Pen3P pénètre dans les cellules, mais n'est pas adressé au noyau. La séquence Pen3P initialement décrite dans DEROSSI et al. [J. Biol. Chem., 271, 30, 18188-18193, (1996)] est RQPKIWFPNRRKPWKK (SEQ ID NO: 7)

La séquence du peptide biotinylé NES-Pen3P est la suivante :
Biot-QSLAQELGLNERQPKIWFPNRRKPWKK-COOH (SEQ ID NO: 8)

Les peptides biotinylés Penet, NES-Penet, Pen3P et NES-Pen3P sont ajoutés, à une concentration de 100 µM, à des cultures de cellules MDCK. Après incubation durant deux heures à 4°C, les cellules sont rincées avec du milieu prérefroidi à 4°C contenant du NaCl à une concentration de 0,5M ; elles sont ensuite immédiatement fixées à l'aide d'une solution de paraformaldéhyde 4% (5 minutes à température ambiante) ou, alternativement, elles subissent une chasse à 37°C avec du milieu de culture dépourvu de peptide durant 30 minutes avant fixation au paraformaldéhyde. Elles sont ensuite perméabilisées durant 5 min à -20°C avec du méthanol, et préincubées une heure dans du PBS contenant 5% de sérum de veau foetal. Les peptides sont révélés à l'aide d'un complexe streptavidine-fluorescéine. La fluorescence est ensuite analysée en microscopie confocale

Le peptide Penet entre à 44°C : on observe à cette température un marquage cytoplasmique diffus, et un marquage nucléaire plus intense, qui s'accentue après passage à 37°C.

Le peptide NES-Penet entre à 4°C ; on observe à cette température un marquage diffus, sans localisation cytoplasmique ou nucléaire notoire. Après passage à 37°C on observe une forte association du marquage à des vésicules intra-cytoplasmiques qui correspondent probablement à un compartiment de sécrétion non post-golgien (Cf. Exemple 2).

Le peptide Pen3P entre à 4°C ; comme dans le cas du peptide NES-Penet, on observe à cette température un marquage cytoplasmique et nucléaire diffus. En revanche, contrairement au peptide NES-Penet ce marquage n'est pas modifié à 37°C.

Le peptide NES-Pen3P entre à 4°C ; comme dans le cas du peptide NES-Penet, on observe à cette température un marquage cytoplasmique et nucléaire diffus. A 37°C on observe une forte association du marquage à des vésicules intra-cytoplasmiques, semblable à celle observée pour NES-Penet.

D'autre part, le passage des peptides à travers un épithélium à jonctions serrées a été évalué en utilisant le dispositif décrit à l'exemple 2 ci-dessus.

Les peptides biotinylés (10 µM) sont incubés dans le compartiment basolatéral, dans les conditions décrites ci-dessus, pendant 30 minutes à 37°C. A la fin de l'incubation, les contenus du compartiment basolatéral et du compartiment apical, et celui des cellules sont analysés comme décrit à l'exemple 2.

On observe une pénétration des peptides Pen3P et NES-Pen3P dans les cellules, et un très faible passage du peptide Penet3P dans le compartiment apical ; en revanche le passage du peptide NES-Penet3P dans le compartiment apical est beaucoup plus important.

Ces résultats montrent que l'adressage à un compartiment de sécrétion, et le passage à travers un épithélium à jonction serrées ne nécessitent pas l'adressage nucléaire préalable des peptides.

## Revendications

1. Peptide comprenant un domaine de transduction dérivé de la troisième hélice d'un homéodomaine et une séquence d'export nucléaire, pour l'utilisation comme vecteur de transport d'une molécule d'intérêt diagnostique ou thérapeutique à travers un épithélium à jonctions serrées.

2. Peptide pour l'utilisation selon la revendication 1, **caractérisé en ce que** ledit épithélium à jonctions serrées est la barrière hémato-méningée ou le plexus choroïde.

3. Peptide pour l'utilisation selon une quelconque des revendications 1 ou 2, **caractérisé en ce que** ladite séquence d'export nucléaire est une séquence d'export nucléaire riche en leucine.

4. Peptide pour l'utilisation selon la revendication 3, **caractérisé en ce que** ladite séquence d'export nucléaire est une séquence d'export nucléaire d'une protéine à homéodomaine.

5. Peptide pour l'utilisation selon la revendication 4, **caractérisé en ce que** ladite séquence d'export nucléaire comprend au moins la séquence peptidique AQELGLNESQI.

6. Peptide pour l'utilisation selon la revendication 5, **caractérisé en ce que** ladite séquence d'export nucléaire comprend au moins la séquence peptidique SLAQELGLNERQIKI.

7. Peptide pour l'utilisation selon une quelconque des revendications 1 à 6, **caractérisé en ce que** la molécule d'intérêt diagnostique ou thérapeutique à transporter est choisie parmi les acides nucléiques, les polypeptides, les peptides/acides nucléiques, et les analogues de nucléotides.

## Patentansprüche

1. Peptid, umfassend eine Transduktionsdomäne, die von der dritten Helix einer Homeodomäne abgeleitet ist, und eine Kernexportsequenz, zur Verwendung als Transportvektor eines diagnostischen oder therapeutischen Moleküls von Interesse durch ein Epithel mit Tight Junctions.

2. Peptid zur Verwendung gemäß Anspruch 1, **dadurch gekennzeichnet, dass** das Epithel mit Tight Junctions die Blut-Hirn-Schranke oder der Plexus choroideus ist.

3. Peptid zur Verwendung gemäß einem der Ansprüche 1 oder 2, **dadurch gekennzeichnet, dass** die Kernexportsequenz eine Kernexportsequenz ist, die reich an Leucin ist.

4. Peptid zur Verwendung gemäß Anspruch 3, **dadurch gekennzeichnet, dass** die Kernexportsequenz eine Kernexportsequenz eines Homeodomänenproteins ist.

5. Peptid zur Verwendung gemäß Anspruch 4, **dadurch gekennzeichnet, dass** die Kernexportsequenz wenigstens die Peptidsequenz AQELGLNESQI umfasst.

6. Peptid zur Verwendung gemäß Anspruch 5, **dadurch gekennzeichnet, dass** die Kernexportsequenz wenigstens die Peptidsequenz SLAQELGLNERQIKI umfasst.

7. Peptid zur Verwendung gemäß einem der Ansprüche 1 bis 6, **dadurch gekennzeichnet, dass** das diagnostische oder therapeutische Molekül von Interesse, das zu transportieren ist, ausgewählt ist aus Nucleinsäuren, Polypeptiden, Peptiden/Nucleinsäuren und den Nucleotidanaloga.

## Claims

1. Peptide comprising a transduction domain derived from the third helix of a homeodomain, and a nuclear export signal, for use as a vector for transporting a molecule of diagnostic or therapeutic interest through an epithelium having tight junctions.

2. Peptide for the use according to claim 1, **characterised in that** the said epithelium having tight junctions is the blood-brain barrier or the choroid plexus.

3. Peptide for the use according to either of claims 1 and 2, **characterised in that** the said nuclear export signal is a leucine-rich nuclear export signal.

4. Peptide for the use according to claim 3, **characterised in that** the said nuclear export signal is a nuclear export signal belonging to a homeodomain protein.

5. Peptide for the use according to claim 4, **characterised in that** the said nuclear export signal comprises at least the peptide sequence AQELGLNESQI.

6. Peptide for the use according to claim 5, **characterised in that** the said nuclear export signal comprises at least the peptide sequence SLAQELGLNERQIKI.

7. Peptide for the use according to any one of claims 1 to 6, **characterised in that** the molecule of diagnostic or therapeutic interest to be transported is selected from the nucleic acids, the polypeptides, the nucleic peptides/acids, and the nucleotide analogues.


### SEQUENCE LISTING

<110> CNRS
   Joliot, Alain
   Dupont, Edmond
   Prochiantz, Alain
<120> Vecteurs de transport à travers un épithélium à jonctions serrées
<130> MJPcb644/44
<150> FR 0014945
   <151> 2000-11-20
<160> 8
<170> PatentIn version 3.1
<210> 1
   <211> 18
   <212> PRT
   <213> Artificial Sequence
<220>
   <223> Synthetic peptide
<400> 1
   Lys Leu Ala Leu Lys Leu Ala Leu Lys Ala Leu Lys Ala Ala Leu Lys
   1               5                   10                  15
   Leu Ala
<210> 2
   <211> 11
   <212> PRT
   <213> Artificial Sequence
<220>
   <223> Synthetic peptide
<400> 2
   Ala Gln Glu Leu Gly Leu Asn Glu Ser Gln Ile
   1               5                   10
<210> 3
   <211> 15
   <212> PRT
   <213> Artificial Sequence
<220>
   <223> Synthetic peptide
<400> 3
   Ser Leu Ala Gln Glu Leu Gly Leu Asn Glu Arg Gln Ile Lys Ile
   1               5                   10                  15
<210> 4
   <211> 27
   <212> PRT
   <213> Artificial Sequence
<220>
   <223> Synthetic peptide
<400> 4
   Gln Ser Leu Ala Gln Glu Leu Gly Leu Asn Glu Arg Gln Ile Lys Ile
   1               5                   10                  15
   Trp Phe Gln Asn Arg Arg Met Lys Trp Lys Lys
               20                  25
<210> 5
   <211> 16
   <212> PRT
   <213> Artificial Sequence
<220>
   <223> Synthetic peptide
<400> 5
   Gln Ser Leu Ala Gln Glu Leu Gly Leu Asn Glu Arg Gln Ile Lys Ile
   1               5                   10                  15
<210> 6
   <211> 16
   <212> PRT
   <213> Artificial Sequence
<220>
   <223> Synthetic peptide
<400> 6
   Arg Gln Ile Lys Ile Trp Phe Gln Asn Arg Arg Met Lys Trp Lys Lys
   1               5                   10                  15
<210> 7
   <211> 16
   <212> PRT
   <213> Artificial Sequence
<220>
   <223> Synthetic peptide
<400> 7
   Arg Gln Pro Lys Ile Trp Phe Pro Asn Arg Arg Lys Pro Trp Lys Lys
   1               5                   10                  15
<210> 8
   <211> 27
   <212> PRT
   <213> Artificial Sequence
<220>
   <223> Synthetic peptide
<400> 8
   Gln Ser Leu Ala Gln Glu Leu Gly Leu Asn Glu Arg Gln Pro Lys Ile
   1               5                   10                  15
   Trp Phe Pro Asn Arg Arg Lys Pro Trp Lys Lys
               20                  25